# EUROPEAN PATENT APPLICATION

(11) **EP 1 563 834 A1**
(43) Date of publication of application: **17.08.2005**
(21) Application number: 03775874.5
(22) Date of filing: 25.11.2003
(51) Int. Cl.: A61K 9/51, A61K 47/42, A61K 48/00, A61K 35/72, C12N 15/88, C07K 14/02, C07K 16/08

(54) **HOLLOW NANOPARTICLES OF PROTEIN AND DRUG USING THE SAME**

(30) Priority: 22.11.2002 JP 2002339925
(71) Applicant: Japan Science and Technology Agency, Kawaguchi-shi, Saitama 332-0012 (JP)
(72) Inventor: KURODA, Shunichi, Suita-shi, Osaka 565-0872 (JP); TANIZAWA, Katsuyuki, Toyono-gun, Osaka 563-0214 (JP); KONDO, Akihiko, Kobe-shi, Hyogo 657-0015 (JP); UEDA, Masakazu, Shinjuku-ku, Tokyo 162-0837 (JP); SENO, Masaharu, Okayama-shi, Okayama 703-8273 (JP)
(74) Representative: Bentz, Jean-Paul
(86) International application number: PCT/JP2003/015003
(87) International publication number: WO 2004/047812

(57) **Abstract**

The subject invention is hollow nanoparticles that comprise particle-forming first proteins (e.g. hepatitis B virus surface-antigen protein), containing a bio-recognizing molecule for recognizing a specific cell, wherein at least one of the first proteins interacts with a second protein (e.g. hepatitis B virus core-antigen protein) forming a capsid structure. With this structure, the present invention provides hollow nanoparticles, that allow transfer of a substance to a specific cell or tissue, and can be manufactured with stable productivity. The present invention further provides a drug made of the hollow nanoparticles.

## Description

### TECHNICAL FIELD

The present invention relates to hollow nanoparticles, that allow a disease-treating target-cell-substance to be encapsulated therein. The invention particularly relates to a drug allowing a disease-treating-target-cell substance encapsulated therein to be transferred to a specific cell or tissue.

### BACKGROUND ART

In the field of medicine, there has been active research on drugs that directly and effectively act on the affected area without causing serious side effects. One area of active research is a method known as a drug delivery system (DDS), in which active ingredients of drugs or other substances are specifically delivered to a target cell or tissue, where they can exhibit their effects.

One known example of conventional method of sending genes to cells is so-called a gene transfer method. In this method, genes encoding the protein are incorporated into an expression vector, and this expression vector is transferred to the target cell by an electroporation method or the like. The transferred vector is expressed in the cell to be the protein functioning to the drug.

Examples of Such techniques can be found in Patent Document 1:Pumphlet of International Publication No. 01/64930, Non-Patent-Document 1: "*Journal of Biological Chemistry*" (US), 267th Issue, 3rd Issue, p.1953-1961, Non-Patent-Document 2: "*Gene*" (Netherlands), 1991, 106th Issue p.143-149, Non-Patent-Document 3, "*Vaccine*" (England) 2001, 19th Issue, p.3154-3163, Non-Patent-Document 4: "*Journal of Virology*" (US), 1994, 68th Issue, p.1643-1650, and Non-Patent-Document 5: "*VIROLOGY*" (US), 1997, 228th Issue, p.115-120 etc.

However, none of the conventional gene transfer methods is sufficient to specifically transfer protein drugs to a target cell/tissue.

Under these circumstances, the inventors of the present invention have previously proposed a method of specifically and safely delivering and transferring various substances (including genes, proteins, compounds) into a target cell or tissue, using hollow nanoparticles of a protein that has the ability to form particles and has incorporated bio-recognizing molecules. However, there has been a need to develop this method to ensure stable production of the hollow nanoparticles.

The present invention was made in view of the foregoing problems, and an object of the invention is to provide hollow protein nanoparticles that allow a substance to be specifically transferred to a target cell or tissue, and can be manufactured with stable productivity. The present invention further provides a drug made of the hollow nanoparticles in which target-cell-substance is encapsulated.

### DISCLOSURE OF INVENTION

As a result of intensive study, the inventors of the present invention accomplished the present invention by successfully coexpressing a particle-forming protein with a protein forming a capsid structure. This coexpression method significantly increases the productivity of particles.

Specifically, the hollow nanoparticles of the present invention comprise particle-forming first proteins, containing a bio-recognizing molecule for recognizing a specific cell, wherein at least one of the first proteins interacts with a second protein forming a capsid structure.

Examples of the "particle-forming first protein" include hepatitis B virus surface-antigen protein. When expressed in the eukaryotic cell, this particle-forming protein is expressed on the endoplasmic reticulum as a membrane protein and accumulates thereon before it is released as particles. The "capsid structure" refers to shells of protein that cover and protect virus genomes of virus particles. Examples of the "second protein forming a capsid structure" include hepatitis B virus core-antigen protein. When coexpressed with the first protein, the second protein interacts with the first protein, and encourages particle formation. The "interaction" here refers to an effect caused by direct contact of the first protein and the second protein. The effect can be obtained with a single first protein directly in contact with a second protein; however, a larger number is more preferable. Further, when interact with the first protein, the second protein does not necessarily have to be encapsulated in the first protein but may exist outside the first protein. With the interaction, the particle formation of the first protein is encouraged, thus increasing particle forming rate. Further, by encapsulating a target-cell substance in the particles, the protein may be used as a drug.

The hollow nanoparticles may be formed by transferring a gene encoding the first protein and a gene encoding the second protein to a single eukaryotic cell (e.g. Yeast) by separate vectors, so that the respective genes are coexpressed in the eukaryotic cell.

The gene encoding the second protein is preferably transferred by a vector having an Aureobasidin A-sensitive gene (e.g. pAUR123: product of TAKARA).

The particles obtained by coexpressing the hepatitis B virus surface-antigen protein and the hepatitis B virus core-antigen protein recognize hepatocyte cells, thus transferring a substance encapsulated therein specifically to the hepatocyte cells. Therefore, by encapsulating a disease-treating substance (such as a gene) in the particles, the protein functions as a drug that specifically and effectively acts on hepatocytes.

Further, by modifying the hepatitis B virus surface-antigen protein to lack the original infectivity to hepatocytes and to display a growth factor or an antigen before formed as particles, the resulting particles will be able to specifically transfer the substance encapsulated therein to other target cells or tissues than hepatocytes. For example, by modifying the protein to display a beta-cellulin (BTC), the protein will identify pancreas β cells, thus specifically delivering substances encapsulated in the particles to the pancreas β cells. Similarly, by modifying the protein to display a basic fibroblast growth factor (bFGF), the protein will identify vascular endothelium or hypophysis cells, thus specifically delivering substances encapsulated in the particles to the vascular endothelium or hypophysis cells.

Such a modified hepatitis B virus surface-antigen protein, like the BTC displaying hepatitis B virus surface-antigen protein or the bFGF displaying hepatitis B virus surface-antigen protein has severe difficulty in particle forming when it is expressed alone; however, by coexpressing with the second protein forming the capsid structure, the particle forming rate will be significantly increase, thus providing a certain effect.

The drug of the present invention, in which a disease-treating substance is encapsulated in hollow nanoparticles, realizes selective and effective treatment with respect to specific cells or tissues, by a convenient method, such as intravenous injection. The invention is a great leap forward from conventional gene therapy in that it does not require a large-amount of drug administration or any surgical operation, and that the risk of side effect is greatly reduced.

A disease treating method of the present invention uses the foregoing drug of the present invention.

For a fuller understanding of the nature and advantages of the invention, reference should be made to the ensuing detailed description taken in conjunction with the accompanying drawings.

### BRIEF DESCRIPTION OF DRAWINGS

Figure 1 is an explanatory view showing modification of a bio-recognizing molecule of HBsAg L particles according to one embodiment of the present invention.
Figure 2(a) is a schematic diagram showing HBcAg L protein that forms the hollow nanoparticles of one embodiment of the present invention, Figure 2(b) is a capsid structure of the HBcAg, and Figure 2(c) shows hepatitis B virus having the capsid structure.
Figure 3(a) is a drawing showing a vector containing a gene encoding HBsAg L protein, used for producing the hollow nanoparticles of one embodiment of the present invention, and Figure3 (b) shows a vector containing a gene encoding HBcAg L protein.
Figure 4 is a drawing showing western blotting of BTC displaying HBsAg L particles and bFGF displaying HBsAg L particles.
Figure 5 is a drawing showing antigenicity of HBsAg L particles, that are hollow nanoparticles of one embodiment of the present invention, obtained by coexpressing wild-type HBsAg L protein and HBcAg.
Figure 6 is a drawing showing antigenicity of EGF displaying HBsAg L particles, that are hollow nanoparticles of one embodiment of the present invention, obtained by coexpressing EGF displaying HBsAg L protein and HBcAg.
Figure 7 is a drawing showing antigenicity of Null-type HBsAg L particles, that are hollow nanoparticles of one embodiment of the present invention, obtained by coexpressing Null-type HBsAg L protein and HBcAg.
Figure 8 is a drawing showing antigenicity of BTC displaying HBsAg L particles, that are hollow nanoparticles of one embodiment of the present invention, obtained by coexpressing BTC displaying HBsAg L protein and HBcAg.
Figure 9 is a drawing showing antigenicity of bFGF displaying HBsAg L particles, that are hollow nanoparticles of one embodiment of the present invention, obtained by coexpressing bFGF displaying HBsAg L protein and HBcAg.
Figure 10 is a drawing showing antigenicity of BTC displaying HBsAg L particles and bFGF displaying HBsAg L particles of one embodiment of the present invention, in which the antigenicity is shown by a relative value when assuming that the antigenicity of the particles formed by single expression of wild-type HBsAg L protein = 100.

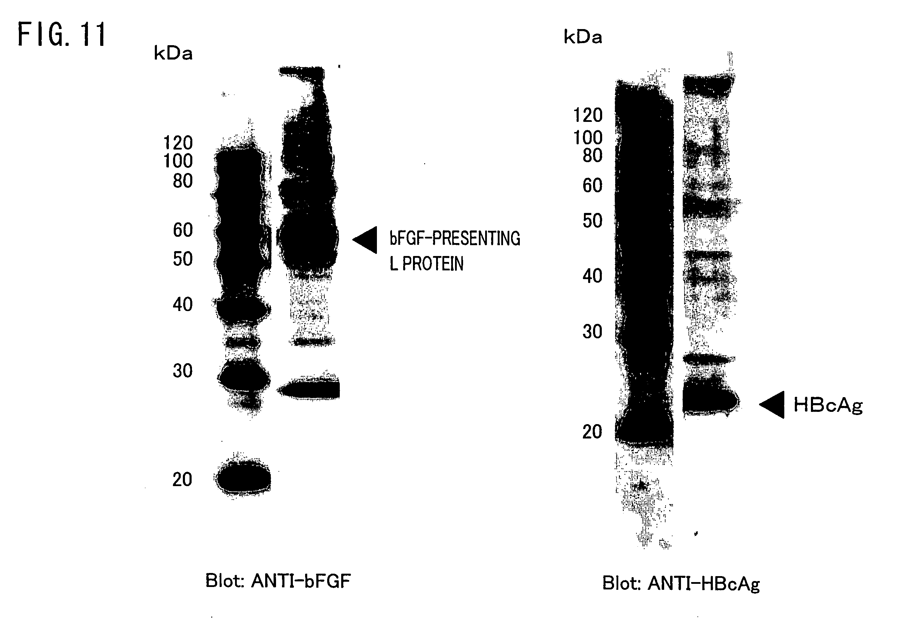

Figure 11 is a drawing showing western blotting of bFGF displaying HBsAg L particles of one embodiment of the present invention.
Figure 12(a) is a drawing showing a CsCl density-gradient centrifugation tube of a result of CsCl density-gradient centrifugation of bFGF displaying HBsAg L particles of one embodiment of the present invention, and Figure 12(b) shows antigenicity for each fraction after the CsCl density-gradient centrifugation.
Figure 13(a) is a drawing showing a result of western blotting of bFGF displaying HBsAg L particles of one embodiment of the present invention after proteinase treatment, using anti-L protein antigen, Figure 13(b) shows western blotting of the same particles using anti-bFGF antigen, and Figure 13(c) shows western blotting of the same particles using anti-HBcAg antigen.
Figure 14(a) is a drawing showing a CsCl density-gradient centrifugation tube of a result of CsCl density-gradient centrifugation of BTC displaying HBsAg L particles of one embodiment of the present invention, Figure 14(b) shows antigenicity for each fraction after the CsCl density-gradient centrifugation, and Figure 14(c) shows a result of western blotting of a crude extract or a fraction of CsCl density-gradient centrifugation.
Figure 15(a) is a drawing showing HBsAg L particles of one embodiment of the present invention, in which HBcAg externally interacts with HBsAg L protein, and Figure 15(b) a drawing a state where HBcAg encapsulated in HBsAg L particles interacts with HBsAg L protein.

### BEST MODE FOR CARRYING OUT THE INVENTION

The present invention discloses hollow nanoparticles made of a first protein with particle-forming ability. At least one of the first proteins interacts with a second protein forming a capsid structure. By incorporating a bio-recognizing molecule (molecule that recognizes a specific cell) to the first protein with the particle-forming ability, the drug becomes capable of specifically delivering a substance to a target cell or tissue. The first protein with the particle-forming ability may be sub viral particles obtained from various viruses. Specific examples of the first protein include hepatitis B virus (HBV) surface-antigen protein (this hepatitis B virus surface-antigen protein may be hereinafter simply referred to as HBsAg protein). In this case, hepatitis B virus core-antigen protein (this may be hereinafter simply referred to as HBcAg protein) may be used as the second protein in the capsid structure.

As to the first protein, particles of a particle-forming protein may be obtained through the protein expression in the eukaryotic cell. Specifically, in eukaryotic cells, the particle-forming protein is expressed on the endoplasmic reticulum as a membrane protein and accumulates thereon before it is released as particles. The eukaryotic cell may be obtained from yeasts, or animals including mammals.

The inventors of the present invention have reported that the expression of HBsAg L protein in recombinant yeast cells produces ellipsoidal hollow particles (hereinafter referred to as HBsAg L particles) with a minor axis of 20 nm and a major axis of 150 nm, with a large number of L proteins embedded in the yeast-derived lipid bilayer membrane (see Non-Patent Document 1). The particles contain no HBV genome and lack the viral function. Therefore, the particles are very safe to the human body. Further, since the particles have on its surface a specific receptor for hepatocytes with high infectivity for HBV hepatocytes, the particles reliably function as a carrier for specifically transferring a substance to hepatocytes.

Therefore, forming the protein particles using recombinant yeasts offers a preferable method of efficiently producing particles from soluble proteins in the yeasts.

As will be described in later Examples, the inventors of the present invention have reported that the production amount of HBsAg L protein particles is significantly increased by expressing the HBsAg L protein with the HBcAg protein. As to the reason of this effect, it may be assumed that the HBcAg interacts with the HBsAg L protein and expedites the particle-formation.

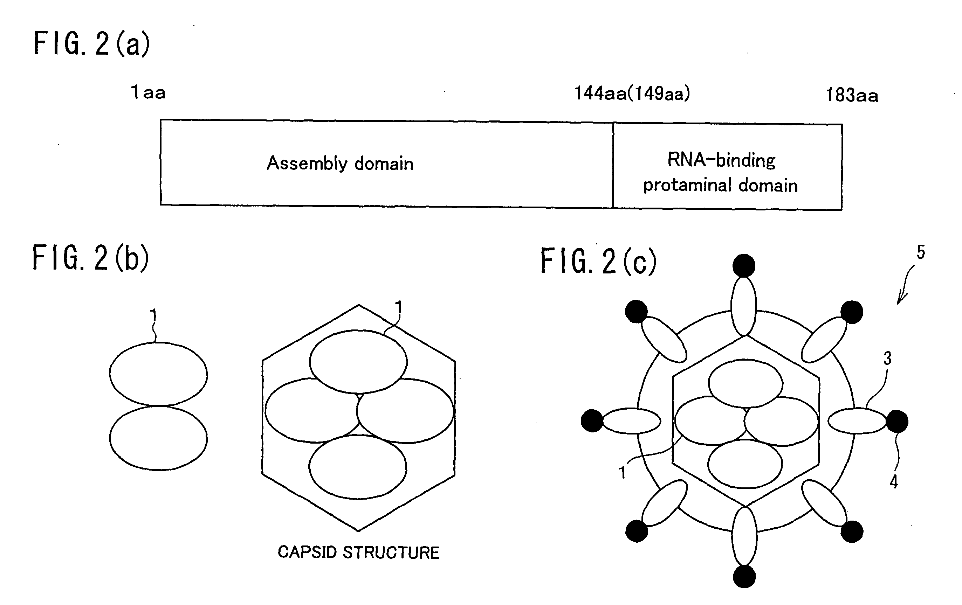

As shown in Figure 2(a), the HBcAg protein is made up of Assembly domain and a RNA-binding protamine domain. The Assembly domain is required to form the capsid structure, meaning that this site is important for formation of the hollow nanoparticles of the present invention. As shown in Figure 2(b), the HBcAg protein has an ability to form the capsid structure by assembling itself; however, they are not always required to be in the capsid state, but the individual molecules may act on the HBsAg L protein separately.

A suitable vector for incorporating the HBsAg L protein and HBcAg protein may be selected from various vectors with different promoters, depending on the purpose. Particularly, when the HBcAg protein is incorporated, the vector for the L protein of HBsAg L protein is preferably a vector containing Aureobasidin-sensitive gene aur1, such as pAUR123 (Product of Takara Corporation), that is known for high effect for expediting the particle formation of HBsAg.

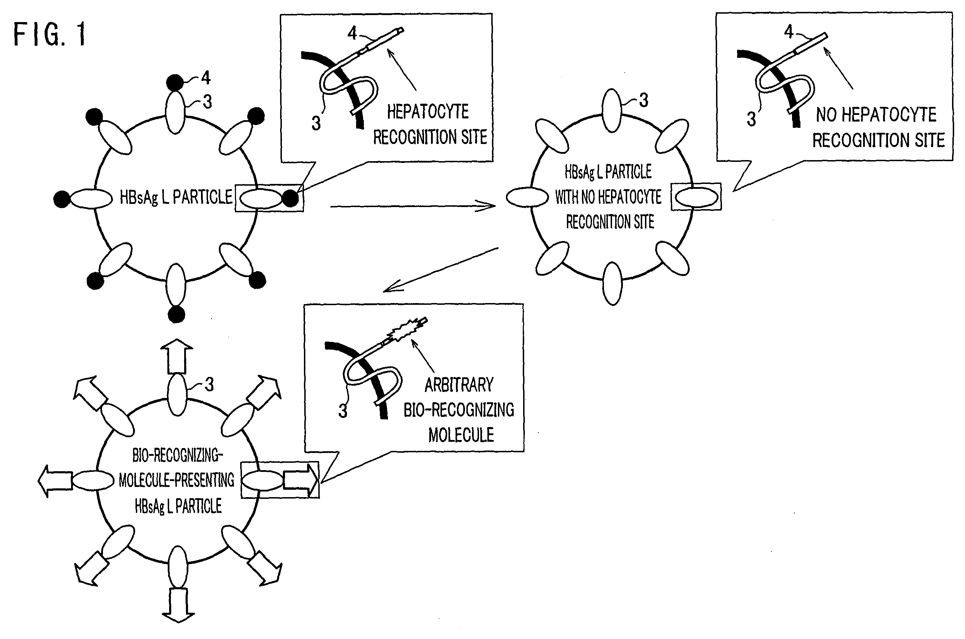

Further, as shown in Figure 1, Hollow protein nanoparticles of the present invention are prepared by modifying a receptor in the surface of particles, that are obtained by the foregoing methods, to a bio-recognizing molecule, as shown in Figure 1. With such modification, the hollow protein nanoparticles can very specifically deliver and transfer a substance to a cell or tissue other than hepatocytes.

The particle-forming first protein is not limited to the hepatitis B virus surface-antigen protein but may be any protein able to form particles. For example, natural proteins from animal cells, plant cells, viruses or fungi, and various types of synthetic proteins may be used. Further, when there is a possibility that, for example, virus-derived antigen proteins may trigger antibody reaction in a target organism, a particle-forming protein with suppressed antigenic action may be used. For example, such a particle-forming protein may be the hepatitis B virus surface-antigen protein modified to suppress its antigenic action, or a protein obtained by modifying the hepatitis B virus surface-antigen protein of the Patent Document 1 through a gene recombinant technology, for example, to have more stable particle structure. Further, another example may be one obtained by adding a growth factor, antibody, or other proteins to a hepatitis B virus surface-antigen protein or a modified hepatitis B virus surface-antigen protein.

Preferable example for the bio-recognizing molecule incorporated in the particle-forming protein include a cell-function-adjusting molecule, such as a growth factor or cytokine, antigens displayed on the cell surface, antigens for specific tissues, molecules for recognizing the cell or tissue, such as a receptor, molecules derived from a virus or a bacteria, an antibody, sugar chain, and lipid. Other example may be an antibody for an EGF receptor and an IL-2 receptor or EGF, specifically displayed on a cancer cell, or a receptor displayed by HBV. Among these, a most suitable one is selected according to the type of target cell or tissue. Note that, the "bio-recognizing molecule" here refers to a molecule that recognizes a specific cell (in other words, a molecule giving the cell-specifying ability to the drug of the present invention). Note that, the bio-recognizing molecule may be either incorporated in the particle-forming first protein or fused (or bonded directly/ indirectly) with the particle-forming first protein.

As described, to add bio-recognizing molecules to the HBsAg L protein particles, a suitable method is modifying the hepatocyte recognition site of the HBsAg L protein to arbitrary bio-recognizing molecules before the protein is expressed. The HBsAg L protein in which the hepatocyte recognition site is thus modified has lower particle-forming ability; however, when interacting with the second protein in the capsid structure, the particle-forming may be significantly improved. This is particularly effective for the HBsAg L protein modified to display BTC or bFGF through the foregoing method. Note that, the second protein may either act within the particles or outside the particles.

The second protein in the capsid structure is not limited to the hepatitis B virus core-antigen protein, but may be any protein can be formed in the capsid structure. Possible examples include natural proteins derived from animal cells, plant cells, viruses, fungus, etc., and various synthetic proteins. The examples also include a hepatitis B virus core-antigen protein modified to improve the ability of forming hollow nanoparticles, and that modified to fix the function.

The hollow nanoparticles of the present invention thus created can effectively deliver a substance specifically to a target cell. For example, by administrating the drug of the present invention, that is made of nanoparticles of the hepatitis B virus surface-antigen protein encapsulating a target-cell-substance, into a living body through intravenous injection, the particles circulate around the body and are lead to the hepatocytes by the hepatocyte-specifying receptor displayed on the particle surface, and finally infect the cell. Consequently, the target-cell-substance is transferred to the hepatocytes, that is, the target-cell-substance is specifically transferred inside the hepar tissue.

The target-cell-substance encapsulated in the hollow protein nanoparticles may be any substance including, for example, genes in the form of DNA or RNA, natural or synthetic proteins, oligonucleotides, peptides, drugs, and natural or synthetic compounds.

These target-cell substances may be encapsulated into the hollow nanoparticles by various methods commonly used in chemical or molecular biological experimental techniques. Some of the preferred examples include an electroporation method, ultrasonic method, simple diffusion method, and a method using charged lipids. Further, when the target-cell-substance is a protein, encapsulation of the target-cell-substance may be performed by fusing the target-cell-substance with the protein having particle-forming ability. An example of the method of fusing the target-cell-substance with the protein having particle-forming ability may be one using a plasmid. In this method, the plasmid contains a gene encoding the hepatitis B virus surface-antigen protein, and also contains a gene encoding the protein drug on the downstream of the gene encoding the hepatitis B virus surface-antigen protein. Using such a plasmid, particles are formed in eukaryotic cell, thereby producing the drug of the present invention in which the hepatitis B virus surface-antigen protein, that forms the particles, is fused with a protein drug.

Note that, the administration of the drug may also be performed through other method than intravenous injection, for example, oral administration, intramuscular administration intraabdominal administration, or subcutaneous administration.

As explained, the drug of the present invention allows a substance to be specifically transported into cells or tissues in vivo or in vitro. Specific transport of the substance into a specific cell or specific tissue may be used as a treatment method of various diseases, or one of the steps in the procedure of the treatment method.

In the following, the present invention will be described in more detail by way of Examples with reference to the attached drawings. It should be appreciated that the present invention is not limited in any ways by the following Examples, and various modifications to details of the invention are possible.

In the following, HBsAg refers to hepatitis B virus surface antigen, that is an envelope protein of HBV; and HBcAg refers to hepatitis B virus core-antigen protein that forms the capsid structure inside the HBV. HBsAg includes S protein made up of 226 amino acids. M protein includes the entire sequence of the S protein with additional 55 amino acids (pre-S2 peptide) at the N-terminus. L protein contains the entire sequence of the M protein with additional 108 amino acids or 119 amino acids (pre-S1 peptide) at the N-terminus. The base sequence of the HBsAg L protein, and its amino-acid sequence are denoted by the sequence numbers 1 and 2, respectively.

The pre-S regions (pre-S1, pre-S2) of HBsAg L protein have important roles in the binding of HBV to the hepatocytes. The Pre-S1 region has a direct binding site for the hepatocytes, and the pre-S2 region has a polymeric albumin receptor that binds to the hepatocytes via polymeric albumin in the blood.

Expression of HBsAg L protein in the eukaryotic cell causes the protein to accumulate as membrane protein on the membrane surface of the endoplasmic reticulum. The L protein molecules of HBsAg agglomerate and are released as particles into the ER lumen, carrying the ER membrane with them as they develop. The particles are hereinafter referred to as hepatitis B virus core-antigen particles (HBsAg L particles).

A large amount of HBsAg L particles thus produced through the foregoing method are hollow sub viral particles that contain no toxic substances. The inventors of the present invention have found the ability of HBsAg L particles to specifically infect human hepatocytes, and using this property, developed a transfer technology of drugs or genes specifically to human hepatocytes.

Further, in view of the fact that the pre-S1 peptide, that is displayed on HBsAg L particle surface, is a site that specifically recognizes human hepatocytes, a modified HBsAg L particles is prepared by replacing the peptide with an arbitrary bio-recognizing molecule, so as to create a system for transferring genes or drugs into an arbitrary organ or cell. Specifically, the replacement is performed by replacing the pre-S 1 peptide with a gene encoding an arbitrary bio-recognizing molecule (ligand, receptor, antibody etc.), since the pre-S1 peptide corresponds to 3rd amino acid residue to the 77th amino acid residue at the N-terminus of the HBsAg L protein. When the HBsAg L protein thus given an arbitrary bio-recognizing molecule is expressed in eukaryotic cells, there are produced HBsAg L particles that display the arbitrary bio-recognizing molecules.

The foregoing method can produce HBsAg L particles with no hepatocyte recognition site (null-type HBsAg L particles), having no ability of specifying human hepatocytes; HBsAg L particles displaying basic fibroblast growth factor (bFGF), specifically infecting vascular endothelium or pituitary grand; HBsAg L particles presenting epidermal growth factor, specifically infecting highly malignant cancer tissues; beta-cellulin (BTC) displaying HBsAg L particles that recognize beta-cellulin (BTC) receptor and specifically infect pancreatic β cells, and antibody-displaying HBsAg L particles in which an antibody with an arbitrary specificity is added to the HBsAg L particles.

However, the modified HBsAg L particles are poor in particle-forming ability. In contrast to the wild-type HBsAg L particles, that express a large rate (40%) of the soluble protein with the use of liquor (sake) brewing yeast, large amount expression is rare for the modified HBsAg L particles, usually only several % of the whole of soluble proteins. Particularly, for the bFGF displaying L protein, only 1% of the soluble protein is expressed.

This is equal to the productivity of a general recombinant protein; however, a larger expression amount is required for the modified HBsAg L particles in use for a drug delivery system. Since the production amount greatly depends on the bio-recognizing molecule added to the HBsAg L particles, it may be assumed that the bio-recognizing molecule is related to the production of the HBsAg L particles. In this view, the unstable expression amount, that will result in inefficient production, can be overcome.

Then, the inventors figured out the reason for low expression rate of the modified HBsAg L particles was that the steric hindrance of the inserted bio-recognizing molecule blocks the passage of the endoplasmic reticulum from the N-terminus in the pre-S region (that the steric hindrance of the inserted bio-recognizing molecule blocks the passage of the N-terminus region of the pre-s region from the endoplasmic reticulum) in the forming process of HBsAg L particles in the yeast fungus.

Meanwhile, according to the Non-Patent Documents 4 and 5, in the forming process of the virus particles, the hepatitis B virus core-antigen protein (HBcAg) interacts with the C-terminus of the L protein pre-S1 region to encourage particle formation in the hepatocytes.

As shown in Figure 2(a), HBcAg is made up 183 amino-acids, that is divided into an assembly domain including 1-144 (or 149) amino acids and a subsequent RNA-binding protamine domain. The assembly domain is a site for forming the capsid structure, and the RNA-binding protamine domain is a site for keeping the structure stable by bonding with the virus genome DNA. As shown in Figure 2(b), the HBcAg has an ability to form the capsid structure by assembling itself. For hepatitis B virus particles, the capsid structure generally exists inside the viral particles, as shown in Figure 2(c). It has been assumed that the capsid structure was securely kept by the virus genome DNA contained in the structure, and internally encourages formation of the virus particles by interacting with the HBsAg L particles. In view of this assumption, an experiment was performed as follows to observe particle forming rate of the HBsAg L particles, that was formed by expressing a HBsAg L protein or a modified HBsAg L protein together with HBcAg. This observation was performed to find out if HBcAg helps formation of HBsAg L particles to ensure stable expression of HBsAg L particles.

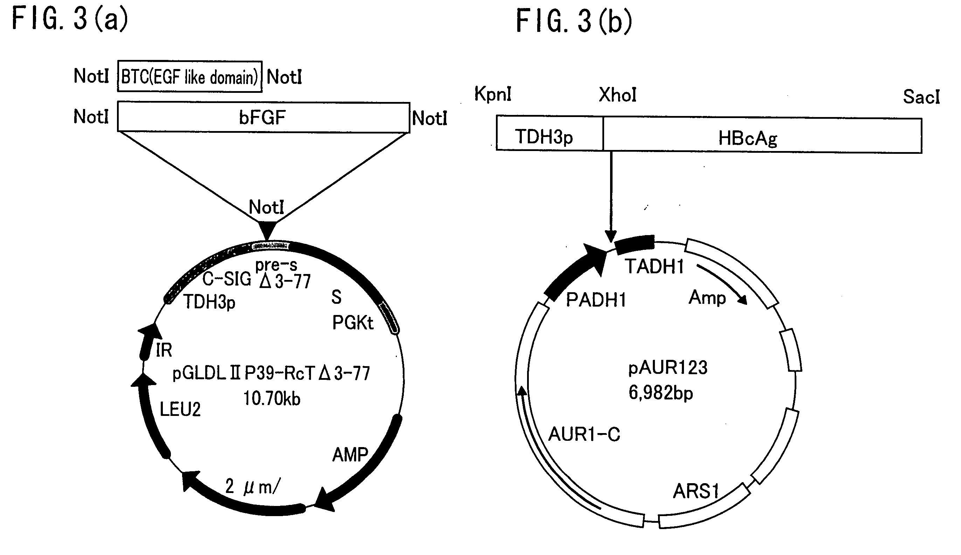

[Example 1] The following explains a preparation method of the modified HBsL protein. As shown in Figure 3(a), the HBsAg L protein-expression-vector according to the Patent Document 1 above, reported by the inventor of the present invention, was expressed in yeast. The vector of pGLDL II P 39-RcT Δ 3-77 was prepared by deleting the pre-S region of pGLDL II P39-RcT(3^{rd} amino residue to 77th amino residue, and inserting genes encoding BTC or bFGF using restriction enzyme NotI. As a result, HBsAg L protein that displays BTC as a bio-recognizing molecule, that is BTC displaying HBsAg L particles, or bFGF displaying HBsAg L particles were obtained.

### [Example 2] Preparation of HBcAg yeast-expression-plasmid

### (2-1) Insertion of promoter TDH3 into a plasmid

A DNA fragment of the promoter TDH3 incorporated in the wild-type HBsAg expression plasmid pRS405-2-LAG was amplified by PCR method. This PCR was performed with a reaction liquid whose composition is shown in Table 1, with the use of primers of sequence numbers 3 and 4, respectively as the primers (+) and (-).

**[Table 1]**

| PCR:HBcAg | |
|---|---|
| 10X LA PCR Buffer II (Mg²⁺free) | 8µl |
| MgCl₂ (25mM) | 8µl |
| dNTP mixture (2.5mM each) | 8µl |
| DNA template (45pg/*µ*l) | 8µl |
| Primer (+) | 0.8µl |
| Primer (-) | 0.8µl |
| TaKaRa LA Taq (5U/*µ*l) | 0.8µl |
| Otsuka distilled water | 45.6µl |
| Total | 80µl |

| PCR:TDH3 | |
|---|---|
| 10X LA PCR Buffer II (Mg²⁺free) | 8µl |
| MgCl₂ (25mM) | 8µl |
| dNTP mixture (2.5mM each) | 8µl |
| DNA template (10ng/*µ*l)) | 8µl |
| Primer (+) | 0.8µl |
| Primer (-) | 0.8µl |
| TaKaRa LA Taq (5U/*µ*l) | 0.8µl |
| Otsuka distilled water | 45.6µl |
| Total | 80µl |

The reaction liquid was divided into four portions, three of which were subjected to PCR reaction in such a manner that: after heating for 30 seconds at 94°C, a set of reaction step comprising 1 minute at 94°C, 1 minute annealing, and 1 minute at 72°C were performed for 30 cycles, before heating at 72°C for five minutes. Three different temperatures: 55°C, 60°C and 65°C were used in the annealing for comparison. After the reaction, it was found that the DNA amplification was done to the same degree for these three types of liquid, and therefore the all PCR products were collected together for TA cloning. Further, for the remaining portion of the reaction liquid, Otsuka distilled water in the same amount was added instead of the DNA template, and was subjected to Annealing at 60°C for negative control.

As shown in Figure 3(b), the DNA fragment obtained through the PCR was conncted to pGEM-T Easy vector (Promega), and after confirmation of the base sequence, cut by restriction enzymes KpnI and XhoI (Takara). The obtained DNA fragment was connected to pAUR123 (Takara), a E Coli/yeast shuttle vector for Aureobasidin A resistant yeast transformation system, by KpnI and XhoI, thus obtaining pAUR123-TDH3. The pAUR123 has Aureobasidin A-sensitive gene aur1, that codes for IPC (inositolphosphorylceramide) synthetic enzyme.

### (2-2) Insertion of HBcAg gene into a plasmid

A DNA fragment of HBcAg was amplified by PCR method using HBV (adr) genome as a template. This HBV genome was a plasmid pHBr330 from Nucleic Acids Res.11 (6), 1747-1757 (1983). Note that, this genome sequence is registered in GenBank accession number D00630. Further, the amino acid sequence of HBcAg gene is registered in the accession number BAA00523.

The PCR was performed with primers of sequence numbers 5 and 6, respectively as the primers (+) and (-). The sequence number 6 in this example contains extra sequence AACGCGTCC (ACGCGT is Mlul site) since it is designed to allow insertion of foreign genes.

The reaction liquid was divided into four portions, three of which were subjected to PCR reaction in such a manner that: after heating for 30 seconds at 94°C, a set of reaction step comprising 1 minute at 94°C, 1 minute annealing, and 1 minute at 72°C were performed for 30 cycles, before heating at 72°C for five minutes. Three different temperatures: 55°C, 60°C and 65°C were used in the annealing for comparison. In this PCR, it was found that the DNA amplification was done to the same degree in two of the three types of liquid, and therefore the PCR products from the two reaction liquids were collected together for TA cloning. Further, for the remaining portion of the reaction liquid, Otsuka distilled water in the same amount was added instead of the DNA template, and was subjected to Annealing at 65°C for negative control.

The DNA fragment obtained through the PCR was connected to pGEM-T Easy vector, and after confirmation of the base sequence, cut by restriction enzymes Xho1 and SacI (Takara). The obtained DNA fragment was connected to pAUR123 TDH3 by Xho1 and SacI, thus obtaining pAUR123-TDH3-HBcAg.

### [Example 3] coexpression of HBcAg and HBsAg in yeast

The HBsAg L protein expression vector of Example 1 and the HBcAg expression vector of Example 2 were cotransformed to each other. In other words, the vectors were transformed to S.cerevisiae AH22R-strain by the following spheroplast method (Albert et al., 1978).

S.cerevisiae AH22R-strain was inoculated on YPDA agar medium (see Table 2 for the composition) and cultivated for a few days at 30°C, and the resulting colony was subjected to preincubation overnight in 50ml YPDA medium (see Table 2 for the composition) at 30°C. After the preincubation, the product was inoculated in 50ml YPDA medium to obtain the condition: OD₆₀₀= 0.05 for the beginning of the main incubation, and then was further cultivated for 8 hours at 30°C until OD₆₀₀ = 0.4-0.8. Thereafter, the culture solution × 4ml is divided into 15ml tubes (IWAKI), and then, the fungus body was collected by centrifugation at 4°C at 2000 rpm for 5 min using a multi-place refrigerated centrifuge (Tommy Corporation), and the collected fungus body was washed with 4ml of solution A (0.1M sodium citrate, 10mM EDTA2 sodium, 1.2M sorbitol, ph5.8). Then, 3.6ml of the solution A and 0.4ml of Zymolyase 100T (Seikagaku Corporation) solution (1.0mg/ml) was added to the washed fungus body, so as to turn them into spheroplast state through incubation at 30°C for an hour.

**[Table 2]**

| YPDA agar medium: | | |
|---|---|---|
| | Polypeptone | 2% |
| | Powder yeast extract | 1% |
| | Adenine hydrosulfate | 0.01% |
| | Glucose | 2% |
| | Agarose | 2% |

| YPDA medium: | | |
|---|---|---|
| | Polypeptone | 2% |
| | Powder yeast extract | 1% |
| | Adenine hydrosulfate | 0.01% |
| | Glucose | 2% |

| Isin-free amino-acid solution(x 10 concentration) | | |
|---|---|---|
| | L-isoleucine | 0.03% |
| | L-valine | 0.15% |
| | L-adenine hydrosulfate | 0.02% |
| | L-arginine salt | 0.02% |
| | L-histidine | 0.02% |
| | L-ricin hydrochloride | 0.03% |
| | L-methionine | 0.02% |
| | L-phenylalanine | 0.05% |
| | L-threonine | 0.2% |
| | L-tryptophan | 0.02% |
| | L-tylosin | 0.03% |
| | L-uracyl | 0.02% |

| Yeast transformation (Y-Tf) agar medium | | |
|---|---|---|
| | Yeast nitrogen base without amino acids | 0.67% |
| | agarose | 2% |
| | sorbitose | 22% |
| | Leucine-Free amino-acid solution(x 10 concentration) | 10% |
| | Glucose | 2% |

| Sodium bicarbonate agar medium | | |
|---|---|---|
| | Yeast nitrogen base without amino acids | 0.67% |
| | Agarose | 2% |
| | sorbitose | 22% |
| | Glucose | 2% |

| Leucine-free amino-acid solution(x 10 concentration) | | |
|---|---|---|
| | Yeast nitrogen base without amino acids | 0.67% |
| | Agarose | 2% |
| | Leucine-free amino-acid solution(× 10 concentration) | 10% |
| | Glucose | 2% |

| Isin-free amino-acid solution (× 10 concentration) | | |
|---|---|---|
| | Yeast nitrogen base without amino acids | 0.67% |
| | Agarose | 2% |
| | Leucine-free amino-acid solution(× 10 concentration) | 10% |
| | Glucose | 2% |
| | Aureobasidin | 0.5 *µ*g/ml |

Thereafter, only the fungus body was collected by centrifugation for 5 min at 20°C at 1900 rpm, using a multi-place refrigerated centrifuge (Tommy Corporation), and was washed with a 4ml solution B (sorbitol 1.2M, calcium chloride 10mM, Tris-HCl (pH7.0)10mM); then, the fungus body was suspended in 0.1ml solution B, and left for 15 minutes at the room temperature after addition of a wild-type or a modified HBsAg L particle-expression-vector × 7µg and pAUR123-TDH3-HBcAg × 5µg. Then, a 2ml solution C (polyethylene glycol 4000 20%, Tris (pH7.0) 10mM, calcium chloride 10mM) was added to the collected fungus body, and further left for 15 minutes. Then, the fungus body was collected by centrifugation for 5 min at 20°C at 1900 rpm, and was suspended in a 0.3ml solution D (sorbitol 1.2M, polypeptone 2%, powder yeast extract 1%, glucose 2%, adenine hydrosulfate 0.01%), before incubated for 20 minutes at 30°C. Thereafter, a 5ml sodium bicarbonate agar medium was added to the fungus suspension kept at 55°C, and was superposed on the Y-Tf agar medium (see Figure 2 for the composition). Then, after cultivation for 6-14 days at 30°C, the resulting colonies were inoculated in a Leucine-free Aureobasidin-A-added SD agar medium (see Table 2 for the composition). Then, cultivation was carried out only for the grown fungus body, and the expressed-strains were screened.

### [Example 4] Measurement of expression amount of coexpression L particles of HBcAg and HBsAg protein

The fungus body thus transformed in Example 3 was subjected to pre-cultivation in a 1ml Hi-Pi medium (see Table 3 for the composition) for three days. The resulting fungus body was collected by centrifugation for 5 min at 4°C at 2000 rpm, using a multi-place refrigerated centrifuge (Tommy Corporation). Then, the medium was replaced with a 1ml 8S5N-P400 medium (see Table 3 for the composition), and the sample was subjected to the main cultivation, that was concussion cultivation, at 30°C for 3-4 days.

**[Table 3]**

| High-Pi medium(1L): | |
|---|---|
| dipotassium hydrogenphosphate | 1.5g |
| potassium iodide | 0.1 mg |
| L-asparagine-1-hydrate | 2g |
| L-histidine | 20mg |
| L-tryptophan | 20mg |
| 1% magnesium sulfate solution | 50ml |
| 1% calcium chloride solution | 33ml |
| Trace solution | 1ml |
| Vitamin solution | 1ml |
| Glucose | 20g |

| 8S5N-P400 medium (1L): | |
|---|---|
| Sucrose | 80g |
| potassium iodide | 0.1 mg |
| L-asparagine-1-hydrate | 5g |
| L-histidine | 300mg |
| potassium chloride | 2g |
| potassium dihydrogenphosphate | 400mg |
| 1% magnesium sulfate solution | 65ml |
| 1% calcium chloride solution | 37ml |
| Trace solution | 5ml |
| Vitamin solution | 2ml |
| Gulcose | 10g |
| 1M Tris-maleic acid solution (pH6.0) | 25ml |

| Trace solution(1 L): | |
|---|---|
| Copper sulfate-5-hydratel | 40mg |
| Iron sulfate-7-hydrate | 250mg |
| Manganese sulfate-4-hydrate | 40mg |
| Zinc sulfate-7-hydrate | 310mg |
| 12-molybdic (VI) ammonium phosphate-3-hydrate | 20mg |

| Vitamine solution(1L): | |
|---|---|
| inositol | 10g |
| thiamine | 200mg |
| pyridoxine | 200mg |
| calcium pantothenate | 200mg |
| niacin | 200mg |
| biotin | 2mg |

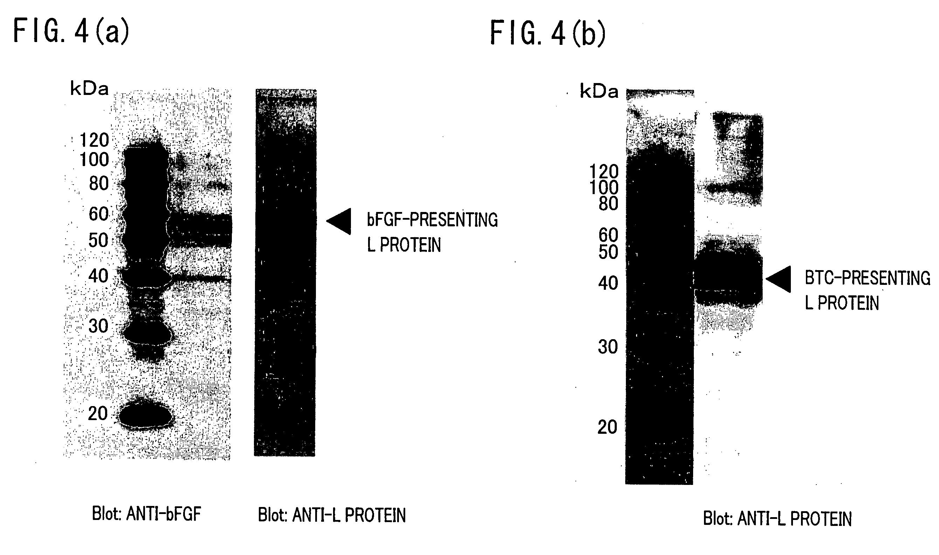

Figure 4(a) shows a result of western blotting of the particles obtained through coexpression of a bFGF displaying HBsAg L protein and HBcAg, with an anti bFGF antibody and anti L protein antibody, and Figure 4(b) shows a result of western blotting of the particles obtained through coexpression of a BTC displaying HBsAg protein L protein and HBcAg, with an anti BTC antibody and anti L protein antibody. As shown by an arrow, the western blotting successfully formed a BTC displaying HBsAg L protein having BTC and L protein, and bFGF displaying HBsAg L particles having bFGF and L protein.

### (A) Observation of expression in 1ml cultivation

Culture solution × 1ml was collected in a 1.5ml micro tube, and the fungus body was collected by centrifugation for 5 min at 4°C at 3000 rpm, and then was suspended in 0.25ml buffering solution A shown in Table 4. Then, glass beads (diameter=0.5mm, BioSpec, Catalog No 11079105) were added before strongly beaten (Vortex) for 25 min at 4°C until the fungus body was destroyed. Further, rough extract was collected by centrifugation for 5 min at 4°C at 10000 rpm, so as to observe the expression amount of modified HBsAg particle and HBcAg, using IMxHBsAg Daina Pack (V2) (HBs antigen in blood detection kit: Dainabot), IMxHBeAg Daina Pack AX.

Note that, the HBeAg is a protein having extra 10 amino acids added to the N-terminus of HBcAg, and the 159 amino acids containing no amino acid residues after 149 amino acid residues at the C-terminus of HBcAg. (the HBeAg is a protein having extra 10 amino acids to the N-terminus of HbcAg and deleted the C-terminus region after 149 amino acid of HbcAg. The HBeAg is the 159 amino acid containing protein.) The HBeAg is secreted from a cell. In contrast to HBcAg, the HBeAg does not have the capsid structure; however, since HBcAg has the same sequence as that of HBeAg except for 10 residues at N-terminus, the expression of HBcAg can be measured with the kit. The measurement however cannot show if the HBcAg is a monomer, a dimmer, or in capsid structure.

Further, when dilution of rough extract is required in using the Daina Pack, PBS of Figure 4 was used.

**[Table 4]**

| Buffering solution A(1L): | |
|---|---|
| Urea | 450.45g |
| disodium hydrogenphosphate-12-hydrate | 24.5g |
| sodium dihydrogenphosphate-2-hydrate | 4.93g |
| EDTA-2Na | 5.58g |
| PMSF | 0.35g |
| Tween80 | 1ml |

| PBS(1L): | |
|---|---|
| sodium chloride | 8g |
| potassium chloride | 0.2g |
| disodium hydrogenphosphate-12-hydrate | 3.63g |
| potassium dihydrogenphosphate | 0.24g |
| (pH7.4) | |

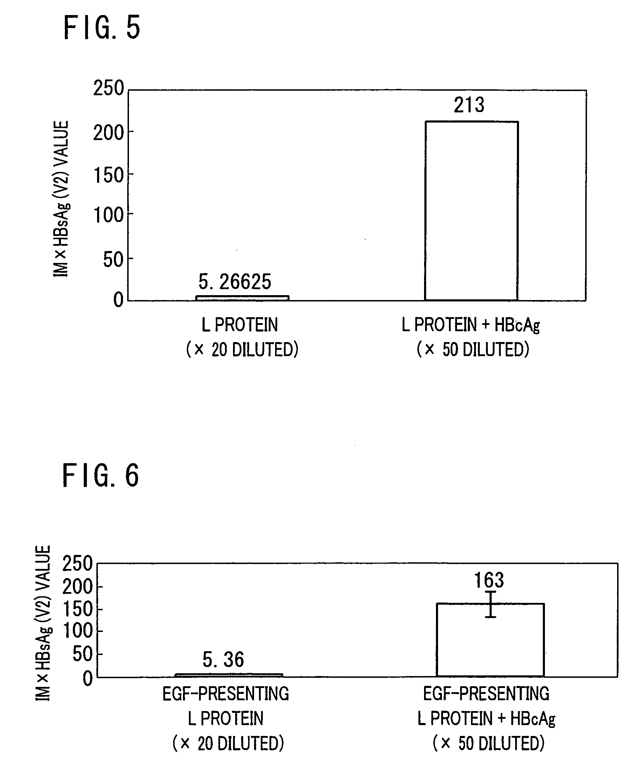

With the foregoing method, a sample, a culture solution (x50 diluted) 1 used for coexpression of wild-type HBsAg L and HBcAg, was created and measured for HBsAg antigenicity. Also, another sample, 8 culture solution (×20 diluted), was prepared for comparative example in the same manner except for addition of HBcAg expression vector, i.e., in which the wild type HBsAg L protein was singly expressed. The result is shown in Figure 5. The vertical axis in Figure 5 shows IMxHBsAg (V2) value, that expresses formation rate of HBsAg particles. As can be seen, the IMxHBsAg (V2) value is 5.27 on average in the culture solution (×20 diluted) for single expression of wild-type HBsAg L, and was 213 in the culture solution (×50 diluted) for coexpression of wild-type HBsAg L and HBcAg, that was significantly high. The result has shown that the particle formation rate can be significantly increased in coexpression of HBsAg L protein and HBcAg.

Figure 6 shows the same experiment but using EGF displaying HBsAg L protein instead of the wild-type HBsAg L protein. Then, in this experiment, the IMxHBsAg (V2) value was measure for the 3 culture solution (×20 diluted) for single expression of EGF displaying HBsAg L protein, and the 17 culture solution (×50 diluted) for coexpression of EGF displaying HBsAg L particles and HBcAg. The value is 5.36 on average in the 3 culture solution (x20 diluted) for single expression of EGF displaying HBsAg L protein, and was 163 in the 17 culture solution (×50 diluted) for coexpression of EGF displaying HBsAg L protein and HBcAg, that was significantly high.

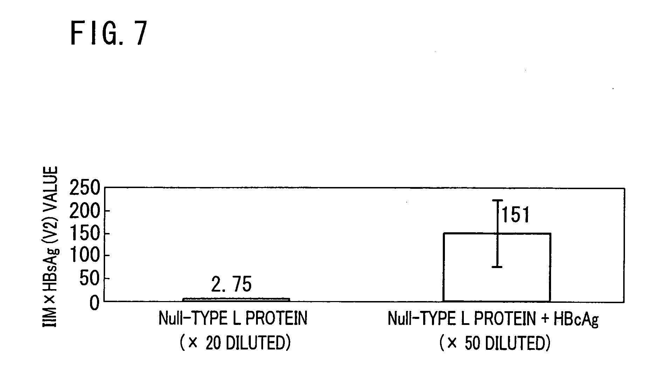

Further, Figure 7 shows the same experiment but using Null-type HBsAg L protein instead of the wild-type HBsAg L protein. Then, in this experiment, the IMxHBsAg (V2) value was measure for the 4 culture solution (×20 diluted) for single expression of Null-type HBsAg L protein, and the 6 culture solution (×100 diluted) for coexpression of Null-type HBsAg L particles and HBcAg. The value is 2.75 on average in the 4 culture solution (×20 diluted) for single expression of Null-type HBsAg L protein, and was 151 in the culture solution (×100 diluted) 6 for coexpression of Null-type HBsAg L protein and HBcAg, that was very high.

### (4-2) Observation of expression in 400ml cultivation

A SD plate with the fungus body in which BTC displaying HBsAg L protein and HBcAg are expressed, and a SD plate with the fungus body in which bFGF displaying HBsAg L protein and HBcAg are expressed were prepared; and the all of fungus bodies of two plates were inoculated in a 50ml Hi-Pi medium to be cultivated for 3 days, followed by another cultivation in a 400ml Hi-Pi medium, and further cultivation in a 8S5N-P400 medium for 3.5 days (86.5 hours). On the day before the last day of cultivation (after 54 hours cultivation in the 8S5N-P400 medium), 1ml of the culture solution was taken from each clone and measured for wet weight. Then, the sample was crushed by glass beads, before measured for expression amount of HBsAg particles with the IMxHBsAg Daina Pack (V2). The ways of crushing and measuring expression amount were the same as those in (4-1) above.

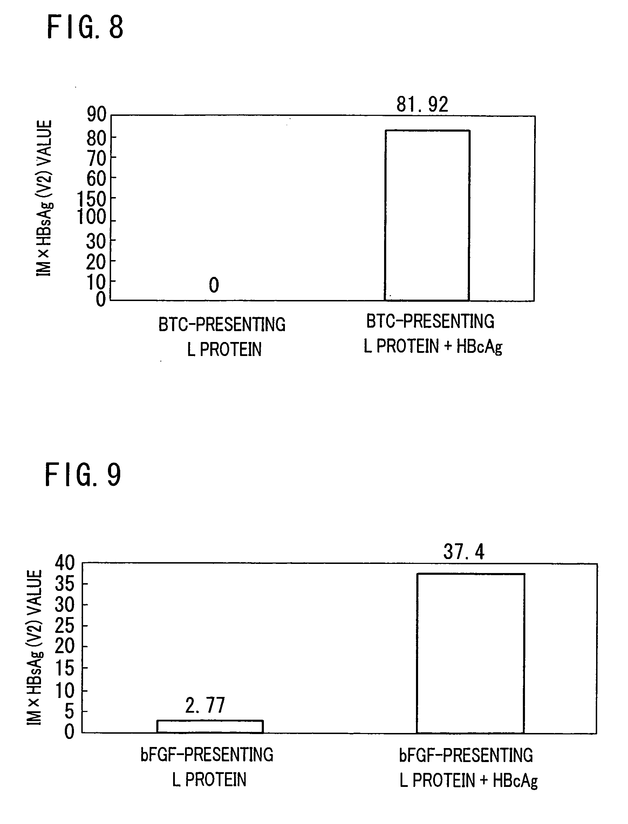

Then, HBsAg antigenicity was measured for the culture solution (×100 diluted) used for coexpression of BTC displaying HBsAg L and HBcAg, and a culture solution (×100 diluted) prepared as a comparative example, that was created in the same manner except for addition of HBcAg expression vector, i.e., in which the BTC displaying HBsAg L protein was singly expressed. The result is shown in Figure 8. As can be seen, the IMxHBsAg (V2) value is 0 in the culture solution for single expression of BTC displaying HBsAg L, that is, there was no expressed fungus body, but was 81.92 in the culture solution for coexpression of HBsAg L particles and HBcAg, that was significantly high.

Figure 9 shows the measurement of HBsAg antigenicity in an experiment with a culture solution (× 100 diluted) for single expression of bFGF displaying HBsAg L protein, and a culture solution (×100 diluted) for coexpression of bFGF displaying HBsAg L particles and HBcAg. The value is 2.77 in the culture solution (×100 diluted) for single expression of HBsAg L protein, but was 37.4 in the culture solution (× 100 diluted) for coexpression of HBsAg L particles and HBcAg, that was significantly high.

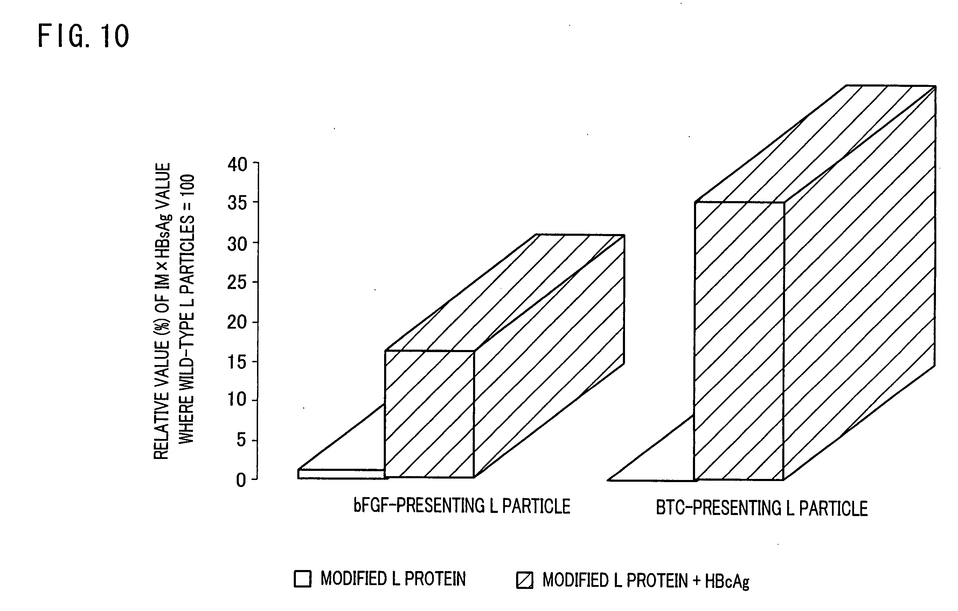

Figure 10 shows the relative value of IMxHBsAg (V2) value with respect to the wild-type HBsAg L particles = 100.

As explained, by coexpressing HBcAg and a modified HBsAg L protein in sake (liquor) yeast, the expression amount of the modified HBsAg L particles are increased by several tens times, that corresponds to several tens % of the whole soluble protein.

### [Example 5] Mass purification of HBsAg L particles

First, through the following method of coexpressing HBcAg and either a L protein or a modified L protein, wild-type HBsAg L particles, Null-type HBsAg L particles, BTC displaying HBsAg particles, bFGF displaying HBsAg particles, and EGF displaying HBsAg particles are prepared for crude samples. Note that, the HBsAg L particles and L protein hereinafter referred to as one of these five types of particles and the L protein forming the particles.

The fungus body was taken from a 400-1000ml of culture solution where the HBcAg and a modified HBsAg L protein are coexpressed, and was suspended in 100ml buffering solution A (see Table 4), followed by crushing for 5 minutes with glass beads (diameter = 0.5mm) using a bead beater, and then left on ice for 5 minutes. This set of processes is repeated three times to completely crush the sample. Then, centrifugation was performed for 10 min at 4°C at 5000 rpm, and 33% (w/v) polyethylene glycol 6000 (Nacalai Tesque) was added to the collected supernatant at a proportion of 75% (v/v) of the solution, followed by cooling on ice for 30 minutes. Further, centrifugation was performed for 10 min at 4°C at 5000 rpm by a centrifuge CX-250 (Tommy Seiko Corporation) with a rotor No. 17N (Tommy Seiko Corporation), and the sediment was collected and suspended in a 15ml buffering solution A to obtain a crude sample.

To purify HBsAg L particles from the crude sample, cesium chloride density-gradient centrifugation, sucrose density-gradient centrifugation, and ultrafiltration were performed.

First, cesium chloride density-gradient centrifugation was performed. This method was performed by carrying out ultracentrifugation of the sample in a cesium chloride solution with density-gradient, so that the HBsAg particles are gathered in that portion of the CsCl solution which has the same density as the floating density of the HBsAg L particles.

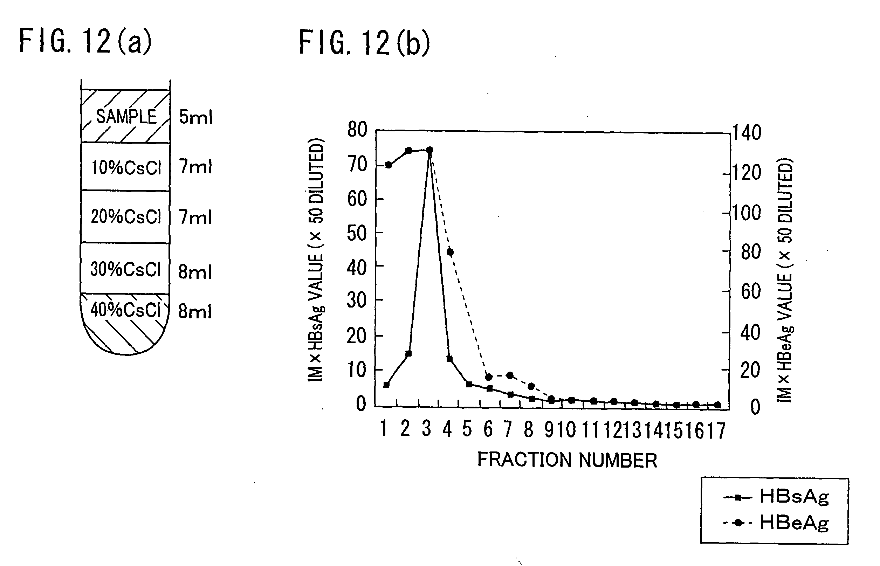

As shown in Figure 12(a), the cesium chloride solution with density-gradient is made of a 40PA tube (Hitachi Koki Corporation) on which 8ml of 40% (w/v) cesium chloride solution, 8ml of 30% (w/v) cesium chloride solution, 7ml of 20% (w/v) cesium chloride solution and 7ml of 10% (w/v) cesium chloride solution were superposed in this order. 5-6ml of the crude sample was further superposed on the liquid surface. Thereafter, by the ultracentrifuge 55P-72H (Hitachi Koki Corporation) with a rotor P28SX (Hitachi Koki Corporation), density-gradient ultracentrifugation was performed at 24000 rpm at 16°C for 12-16 hours.

3-6 tubes having been subjected to the cesium chloride density-gradient centrifugation were divided into 2ml portions from the upper layer, and each portion of the tube was collected as one group to be separately measured by the IMxHBsAg Daina Pack (V2). Then, only the portions where HBsAg L particles were detected were collected to be dialyzed for 3-5 hours at 4°C by PBS containing 15mMEDTA·2Na. The dialyzed solution was subjected to centrifugation for 10 min at 4°C at 5000 rpm by a centrifuge CX-250 with a rotor No. 17N, so as to collect the supernatant.

Next, the supernatant was subjected to sucrose density-gradient centrifugation. This method is performed by carrying out ultracentrifugation of the sample in a sucrose solution with density-gradient, so that the HBsAg particles are precipitated at a speed corresponding to the sedimentation coefficient, and are separated from other molecules different in size and sedimentation coefficient. The sucrose solution with density-gradient is made of a 40PA tube on which 6ml of 50% (w/v) sucrose solution, 5ml of 40% (w/v) cesium chloride solution, 6ml of 30% (w/v) sucrose solution, 5ml of 20% (w/v) sucrose solution, and 5ml of 10% (w/v) sucrose solution were superposed in this order. 8-9ml of the supernatant after the dialysis was further superposed on the liquid surface. Thereafter, by the ultracentrifuge 55P-72H with the rotor P28SX, density-gradient ultracentrifugation was performed at 24000 rpm at 4°C for 8-10 hours.

As in the cesium chloride density-gradient centrifugation, 3-6 tubes having been subjected to the scrose density-gradient centrifugation were divided into 2ml portions from the upper layer, and each portion of the tube was collected as one group to be separately measured by the IMxHBsAg Daina Pack (V2). Then, only the portions where HBsAg L particles were detected were collected.

Finally, the collected portions were dialyzed for overnight at 4°C by PBS, followed by ultrafiltration using ULTRA FILTER UK-200 (Advantec Corporation), to be condensed until the content becomes 10ml.

With the foregoing steps, wild-type HBsAg L particles (with estimated L protein molecular amount = 52kDa), Null-type HBsAg L particles (with estimated Null protein molecular amount = 40.2kDa), BTC displaying HBsAg particles (with estimated BTC fusion L protein molecular amount = 45.7kDa), bFGF displaying HBsAg particles (with estimated bFGF fusion L protein molecular amount = 57.3kDa), and EGF displaying HBsAg particles (with estimated EGF fusion L protein molecular amount = 47.2kDa) were obtained.

### [Example 6] Analysis of physicality of bFGF displaying particles obtained through purification

### (6-1) Analysis by western blotting

Figure 4(a) shows a result of western blotting of the bFGF displaying particles, that has been immunoprecipitated by a S-antibody. The western blotting was performed using a bFGF antibody and a HBcAg protein antibody, so as to find out if the bFGF displaying HBsAg L particles were interacting with the HBcAg protein. As shown by an arrow, the bFGF displaying HBsAg L particles were detected by both the bFGF antigen and the HBcAg protein. Thus, the result has shown that the bFGF displaying HBsAg L particles has the bFGF, HBsAg region, and HBcAg protein. Namely, HBcAg protein caused some effect by interacting with the bFGF displaying HBsAg L particles.

### (6-2) Measurement of density of HBsAg L particles by density-gradient centrifugation

The density of the bFGF displaying HBsAg L particles was measured by cesium chloride density-gradient centrifugation as follows, so as to find out if the bFGF displaying HBsAg L particles contain HBcAg protein.

Figure 12(b) shows the result of cesium chloride density-gradient centrifugation to divide the obtained bFGF displaying HBsAg L particles. This centrifugation was performed in the same manner as that for the purification above. In the graph of Figure 12(b), the IMxHBsAg value and IMxHBeAg value of the solution (×50 diluted) in each portion of CsCl density-gradient sedimentation after the centrifugation are plotted. The portion of CsCl density-gradient sedimentation after the centrifugation is created by dividing the centrifugation product into 17 portions. The peak of the IMxHBsAg value is clearly seen in the fraction 3, i,e., a layer 4ml-6ml away from the upper layer. In this example, the samples are also collected at 2ml intervals from the upper end of the superposed centrifuge tube, as in Figure 12(a), and therefore, it is assumed that the sample of the fraction 3 exists in the 10% CsCl solution. Since the density of the 10% CsCl solution is approximately 1.20g/ml, it is assumed that the density of the bFGF displaying HBsAg L particles is approximately 1.20g/ml. This is similar to the density of wild-type HBsAg L particles, that is 1.21g/ml (see Non-Patent Document 1).

According to Non-Patent Document 2, the density of virion particles, created by coexpressing S, M, L and HBcAg protein, wherein HBcAg protein is encapsulated in HBsAg particles is approximately 1.25g/ml. Therefore, if the HBsAg L particles created by coexpressing L protein and HBcAg protein encapsulates the HBcAg protein therein, it should exist in the portion equal to the density = 1.25g/ml. Accordingly, it has been shown that the bFGF displaying HBsAg L particles with estimated density = about 1.20g/ml does not contain HBcAg protein.

### (6-3) Analysis by proteinase

Further, proteinase treatment was carried out so as to find out if the bFGF displaying HBsAg L particles contain HBcAg protein.

240µl IMx antibody of anti-HBs mouse monoclonal antibody fixing micro particles was added to 720µl purified sample of bFGF displaying HBsAg L particles, thus creating 960µl solution, that was then divided into two tubes. The tubes were immunoprecipitated by being shaken at 4°C for 9 hours, followed by three times washing with a protease inhibitor and a TritonX-100-free lysis buffer. Each sediment was divided into 4 parts, making 8 parts in all. The sediment samples of bFGF displaying HBsAg L particles were thus obtained.

20µl of buffer solution (50mM Tris-HCL, 5mM calcium chloride, pH7.6) containing 10µg/ml P.K. (proteinase K) was added to each of 6 sediments, that were then heated up at 37°C for 5 minutes, 10 minutes, 15 minutes, 20 minutes, 25 minutes and 30 minutes.

For reference, a P.K.-free solution (50mM Tris-HCL, 5mM calcium chloride, pH7.6) was added to the sediments, and only one of them was heated up at 37°C for 30 minutes to be used as Reference sample 2. The one was not be heated was used as Reference sample 1. Further, for another reference, 240µl antibody was added to 720µl PBS to create a 960µl solution, that was subjected to centrifugation. Then, a P.K.-free solution (50mM Tris-HCL, 5mM calcium chloride, pH7.6) was added to the sediment bonded with the 240µl IMx antibody of anti-HBs mouse monoclonal antibody fixing micro particles to be used as Reference sample 3 that was not be heated.

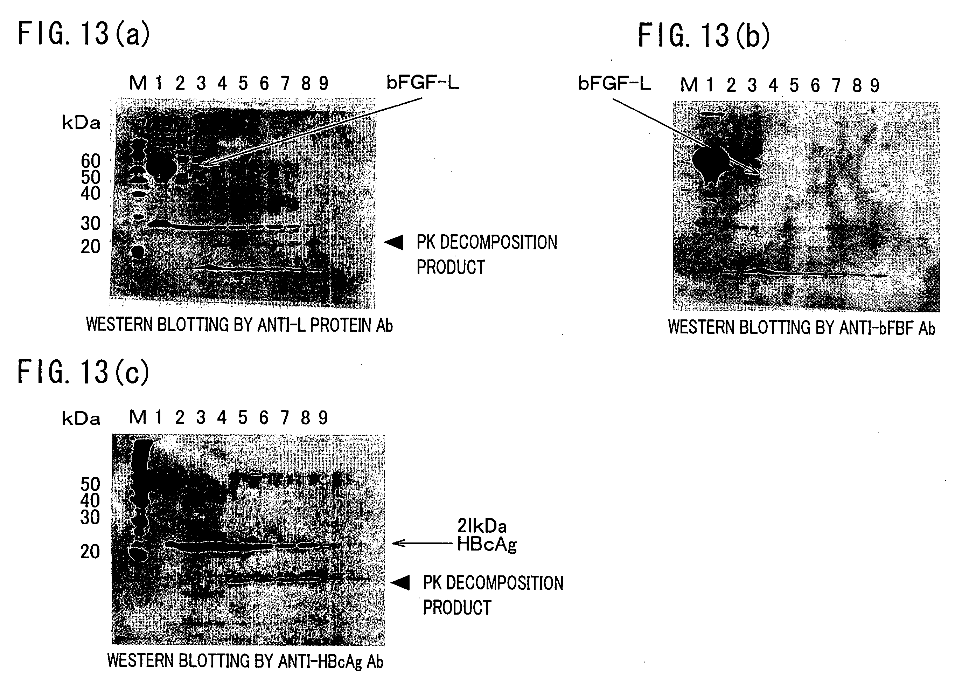

Figure 13 shows the result of western blotting of the samples. Figure 13(a), 13(b) and 13(c) show the western blotting by anti-L protein Ab, by anti-bFGFAb, and by anti-HBcAgAb, respectively. The lanes 1 through 3 have the foregoing Reference Samples3, 1 and 2, respectively, while the lanes 4 through 9 have the samples containing P.K., that were heated for 5 minutes, 10 minutes, 15 minutes, 20 minutes, 25 minutes, and 30 minutes, respectively.

As indicated by the arrows, bands of the bFGF displaying HBsAg L protein are shown in Figures 13(a) and 13(b). The thick arrows point the portion around 20kDa of Figure 13(a) and the portion less than 20kDa of Figure 13(c), where bands that appears to be decomposition products of PK are shown. These bands are not shown in the lanes 1 through 3, but in the lanes 4 through 9.

It is assumed that the band of the decomposition products by PK shown in Figure 13(c) is decomposition product by HBcAg. The band becomes thinner with elapse of proteinase treatment time, and completely disappears after 30 minutes (the lane 9). Namely, HBcAg protein was decomposed by P.K. in a short time.

If the HBcAg is encapsulated in the HBsAg L particles, the HBcAg protein is not externally disposed, and is not easily decomposed by proteinase K. Accordingly, since the result shows that the HBcAg were substantially completely discomposed after 30 minutes heating, HBcAg protein is not encapsulated in bFGF displaying HBsAg L particles.

### [Example 7] Analysis of physicality of BTC displaying particles obtained through purification

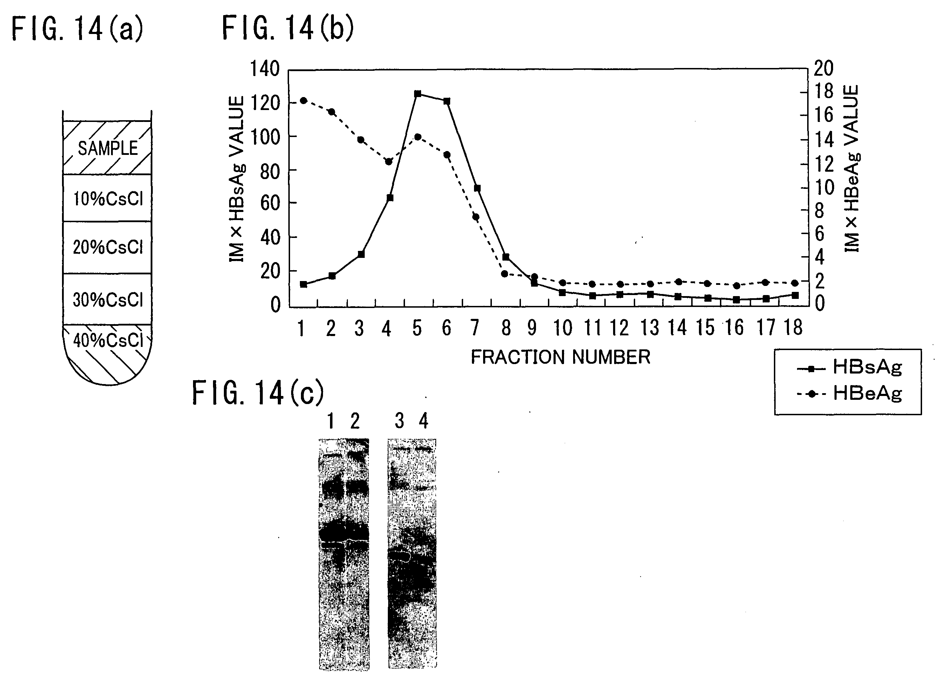

The BTC displaying HBsAg L particles was also subjected to cesium chloride density-gradient centrifugation in the same manner as that for the bFGF displaying L protein. Figure 14 shows the result. In the graph of Figure 14, the IMxHBsAg value and IMxHBcAg value of the solution (×200 diluted) in each portion of CsCl density-gradient sedimentation after the centrifugation are plotted. The peak of the IMxHBsAg value is clearly seen in the fractions 5 and 6. Since the peak exists between the fractions, it is assumed that the BTC displaying HBsAg L protein forms particles. Further, since the peak of IMxHBcAg value and the peak of IMxHBsAg value are partially overlaid, there is a possibility that the BTC displaying HBsAg L particles are partially interact with HBcAg protein.

As shown in Figure 14(a), the samples after the CsCl density-gradient centrifugation are collected at 2ml intervals from the upper end of the superposed centrifuge tube, and therefore, the sample of the fraction 6 exists in the vicinity of the border line of the 10% CsCl solution (density=about 1.20g/ml) and the 20% CsCl solution (density=about 1.27g/ml). The judgment as to weather or not the HBcAg protein is encapsulated in the BTC displaying HBsAg L particles is therefore apparently impossible with this density that was found according to the graph.

The fractions 4 through 7, including the fractions 5 and 6, are collected and were subjected to dialysis by PBS (15mM; EDTA contained), followed by western blotting by anti-HBsAg L particles Ab. Figure 14(c) shows the result of western blotting in the lane 2. The band shown in this example was the same in thickness as the band (lane 1) resulted from western blotting of the crude extract of the BTC displaying HBsAg L particles by anti-HBsAg L particles Ab.

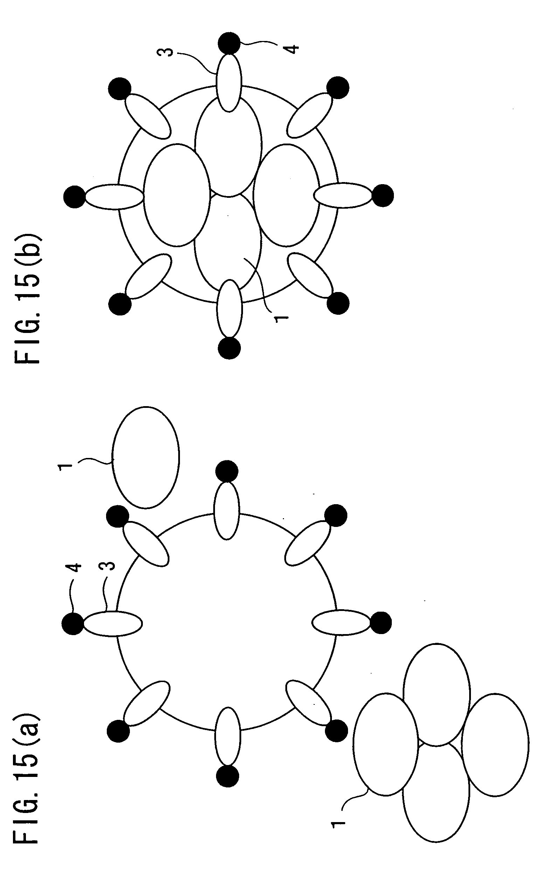

On the other hand, the detection amount of the western blotting product (lane 4) of the sample of lane 2 (dialysis product of the samples of fractions 4 through 7) by anti-HBcAg Ab was much less than the amount of the western blotting product (lane 3) of the crude extract of the BTC displaying HBsAg L particles by anti-HBcAg Ab. That is, in contrast to the BTC displaying HBsAg L particles that exist only in the fractions 4 through 7, HBcAg is dispersed also in other fractions. Accordingly, it is assumed that there is little possibility that the HBcAg protein is encapsulated in the BTC displaying HBsAg L particles as in the example of Figure 15(b), or even when there are any HBcAg protein are encapsulated, the amount is a little. That is, most of the obtained particles of appear to externally interact with HBsAg L protein, as shown in Figure 15(a).

Namely, HBcAg was encapsulated only in a part of the obtained modified HBsAg L particles, and the rest were hollow particles. With this result, it has shown that the hollow nanoparticles of the present invention are suitable in use for carrying a substance for transferring genes etc.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

As explained, the hollow nanoparticles of the present invention provide an effect of efficient particle formation, thus more efficiently producing the particles, while maintaining the ability of transferring a substance specifically to a target cell.

Further, according to the present invention, the particle forming efficiency is kept even after replacement of a bio-recognizing molecule, thus ensuring stable particle formation. Efficient formation of hollow nanoparticles enables stable production of the hollow nanoparticles, allowing the hollow nanoparticles to be used for drugs etc. In this view, the present invention can be used for extensive purposes.

The drug of hollow nanoparticles encapsulating a substance provides a drug enabling selective and effective transfer of a disease-treating target-cell-substance to specific diseased cells or tissues, by a convenient method, such as intravenous injection. The invention is a great leap forward from conventional gene therapy in that it does not require any surgical operation, and that the risk of side effect is greatly reduced. Accordingly, the present invention is suitably used for various purposes in medical-related industry, or in medicinal-drug-related industry etc.

## Claims

1. Hollow nanoparticles that comprise particle-forming first proteins, containing a bio-recognizing molecule for recognizing a specific cell, wherein at least one of the first proteins interacts with a second protein forming a capsid structure.

2. The hollow nanoparticles as set forth in claim 1, wherein the first protein comprises a hepatitis B virus surface-antigen protein.

3. The hollow nanoparticles as set forth in claim 2, wherein the first protein comprises a hepatitis B virus surface-antigen protein whose hepatocyte recognition site is modified to another bio-recognizing molecule.

4. The hollow nanoparticles as set forth in claim 3, wherein the first protein comprises a hepatitis B virus surface-antigen protein whose hepatocyte recognition site is modified to a beta-cellulin or a basic fibroblast growth factor.

5. The hollow nanoparticles as set forth in claims 1 through 4, wherein the second protein comprises a hepatitis B virus core-antigen protein.

6. The hollow nanoparticles as set forth in claims 1 through 5, wherein the hollow nanoparticles are formed by transferring a gene encoding the first protein and a gene encoding the second protein to a single eukaryotic cell by separate vectors, so that the respective genes are coexpressed in the eukaryotic cell.

7. The hollow nanoparticles as set forth in claim 6, wherein the eukaryotic cell is a yeast cell.

8. The hollow nanoparticles as set forth in claim 6 or 7, wherein the gene encoding the second protein is transferred by a vector having an Aureobasidin A-sensitive gene.

9. A drug that is made of the hollow nanoparticles as set form in any one of claims 1 through 8, wherein a target cell substance is encapsulated in the hollow nanoparticles.

10. A disease treating method using the drug as set forth in claim 8.


SEQUENCE LISTING

<110> Japan Science and Technology Agency

<120> Protein hollow nanoparticles and its-derived drugs

<130> P023P08/PCT

<150> JP 2002-339925
<151> 2002-11-22

<160> 6

<170> PatentIn Ver. 2.1

<210> 1
<211> 1218
<212> DNA
<213> Hepatitis B virus

<220>
<221> CDS
<222> (1)..(1218)
atg aga tct ttg ttg atc ttg gtt ttg tgt ttc ttg cca ttg gct gct 48
Met Arg Ser Leu Leu Ile Leu Val Leu Cys Phe Leu Pro Leu Ala Ala
1               5                   10                  15

ttg ggt aag gtt cga caa ggc atg ggg acg aat ctt tct gtt ccc aat 96
Leu Gly Lys Val Arg Gln Gly Met Gly Thr Asn Leu Ser Val Pro Asn

            20                  25                  30

cct ctg gga ttc ttt ccc gat cac cag ttg gac cct gcg ttc gga gcc 144
Pro Leu Gly Phe Phe Pro Asp His Gln Leu Asp Pro Ala Phe Gly Ala
        35                  40                  45

aac tca aac aat cca gat tgg gac ttc aac ccc aac aag gat caa tgg 192
Asn Ser Asn Asn Pro Asp Trp Asp Phe Asn Pro Asn Lys Asp Gln Trp
    50                  55                  60

cca gag gca aat cag gta gga gcg gga gca ttc ggg cca ggg ttc acc 240
Pro Glu Ala Asn Gln Val Gly Ala Gly Ala Phe Gly Pro Gly Phe Thr
65                  70                  75                  80

cca cca cac ggc ggt ctt ttg ggg tgg agc cct cag gct cag ggc ata 288
Pro Pro His Gly Gly Leu Leu Gly Trp Ser Pro Gln Ala Gln Gly Ile
                85                  90                  95

ttg aca aca gtg cca gca gca cct cct cct gcc tcc acc aat cgg cag 336
Leu Thr Thr Val Pro Ala Ala Pro Pro Pro Ala Ser Thr Asn Arg Gln
            100                 105                 110

tca gga aga cag cct act ccc atc tct cca cct cta aga gac agt cat 384
Ser Gly Arg Gln Pro Thr Pro Ile Ser Pro Pro Leu Arg Asp Ser His
        115                 120                 125

cct cag gcc atg cag tgg aat tcc aca aca ttc cac caa gct ctg cta 432
Pro Gln Ala Met Gln Trp Asn Ser Thr Thr Phe His Gln Ala Leu Leu
    130                 135                 140

gat ccc aga gtg agg ggc cta tat ttt cct gct ggt ggc tcc agt tcc 480
Asp Pro Arg Val Arg Gly Leu Tyr Phe Pro Ala Gly Gly Ser Ser Ser
145                 150                 155                 160

gga aca gta aac cct gtt ccg act act gcc tca ccc ata tct ggg gac 528
Gly Thr Val Asn Pro Val Pro Thr Thr Ala Ser Pro Ile Ser Gly Asp
                165                 170                 175

cct gca ccg aac atg gag aac aca aca tca gga ttc cta gga ccc ctg 576
Pro Ala Pro Asn Met Glu Asn Thr Thr Ser Gly Phe Leu Gly Pro Leu
            180                 185                 190

ctc gtg tta cag gcg ggg ttt ttc ttg ttg aca aga atc ctc aca ata 624
Leu Val Leu Gln Ala Gly Phe Phe Leu Leu Thr Arg Ile Leu Thr Ile
        195                 200                 205

cca cag agt cta gac tcg tgg tgg act tct ctc aat ttt cta ggg gga 672
Pro Gln Ser Leu Asp Ser Trp Trp Thr Ser Leu Asn Phe Leu Gly Gly
    210                 215                 220

gca ccc acg tgt cct ggc caa aat tcg cag tcc cca acc tcc aat cac 720
Ala Pro Thr Cys Pro Gly Gln Asn Ser Gln Ser Pro Thr Ser Asn His
225                 230                 235                 240

tca cca acc tct tgt cct cca att tgt cct ggc tat cgc tgg atg tgt 768
Ser Pro Thr Ser Cys Pro Pro Ile Cys Pro Gly Tyr Arg Trp Met Cys
                245                 250                 255

ctg cgg cgt ttt atc ata ttc ctc ttc atc ctg ctg cta tgc ctc atc 816
Leu Arg Arg Phe Ile Ile Phe Leu Phe Ile Leu Leu Leu Cys Leu Ile
            260                 265                 270

ttc ttg ttg gtt ctt ctg gac tac caa ggt atg ttg ccc gtt tgt cct 864
Phe Leu Leu Val Leu Leu Asp Tyr Gln Gly Met Leu Pro Val Cys Pro
        275                 280                 285

cta ctt cca gga aca tca acc acc agc acg ggg cca tgc aag acc tgc 912
Leu Leu Pro Gly Thr Ser Thr Thr Ser Thr Gly Pro Cys Lys Thr Cys
    290                 295                 300

acg att cct gct caa gga acc tct atg ttt ccc tct tgt tgc tgt aca 960
Thr Ile Pro Ala Gln Gly Thr Ser Met Phe Pro Ser Cys Cys Cys Thr
305                 310                 315                 320

aaa cct tcg gac gga aac tgc act tgt att ccc atc cca tca tcc tgg 1008
Lys Pro Ser Asp Gly Asn Cys Thr Cys Ile Pro Ile Pro Ser Ser Trp
                325                 330                 335

gct ttc gca aga ttc cta tgg gag tgg gcc tca gtc cgt ttc tcc tgg 1056
Ala Phe Ala Arg Phe Leu Trp Glu Trp Ala Ser Val Arg Phe Ser Trp
            340                 345                 350

ctc agt tta cta gtg cca ttt gtt cag tgg ttc gta ggg ctt tcc ccc 1104
Leu Ser Leu Leu Val Pro Phe Val Gln Trp Phe Val Gly Leu Ser Pro
        355                 360                 365

act gtt tgg ctt tca gtt ata tgg atg atg tgg tat tgg ggg cca agt 1152

Thr Val Trp Leu Ser Val Ile Trp Met Met Trp Tyr Trp Gly Pro Ser
    370                 375                 380

ctg tac aac atc ttg agt ccc ttt tta cct cta tta cca att ttc ttt 1200
Leu Tyr Asn Ile Leu Ser Pro Phe Leu Pro Leu Leu Pro Ile Phe Phe
385                 390                 395                 400

tgt ctt tgg gta tat att 1218
Cys Leu Trp Val Tyr Ile
                405

<210> 2
<211> 406
<212> PRT
<213> Hepatitis B virus

<400> 2
Met Arg Ser Leu Leu Ile Leu Val Leu Cys Phe Leu Pro Leu Ala Ala
1               5                   10                  15

Leu Gly Lys Val Arg Gln Gly Met Gly Thr Asn Leu Ser Val Pro Asn
            20                  25                  30

Pro Leu Gly Phe Phe Pro Asp His Gln Leu Asp Pro Ala Phe Gly Ala
        35                  40                  45

Asn Ser Asn Asn Pro Asp Trp Asp Phe Asn Pro Asn Lys Asp Gln Trp
    50                  55                  60

Pro Glu Ala Asn Gln Val Gly Ala Gly Ala Phe Gly Pro Gly Phe Thr
65                  70                  75                  80

Pro Pro His Gly Gly Leu Leu Gly Trp Ser Pro Gln Ala Gln Gly Ile
                85                  90                  95

Leu Thr Thr Val Pro Ala Ala Pro Pro Pro Ala Ser Thr Asn Arg Gln
            100                 105                 110

Ser Gly Arg Gln Pro Thr Pro Ile Ser Pro Pro Leu Arg Asp Ser His
        115                 120                 125

Pro Gln Ala Met Gln Trp Asn Ser Thr Thr Phe His Gln Ala Leu Leu
    130                 135                 140

Asp Pro Arg Val Arg Gly Leu Tyr Phe Pro Ala Gly Gly Ser Ser Ser
145                 150                 155                 160

Gly Thr Val Asn Pro Val Pro Thr Thr Ala Ser Pro Ile Ser Gly Asp
                165                 170                 175

Pro Ala Pro Asn Met Glu Asn Thr Thr Ser Gly Phe Leu Gly Pro Leu
            180                 185                 190

Leu Val Leu Gln Ala Gly Phe Phe Leu Leu Thr Arg Ile Leu Thr Ile
        195                 200                 205

Pro Gln Ser Leu Asp Ser Trp Trp Thr Ser Leu Asn Phe Leu Gly Gly

210                 215                 220

Ala Pro Thr Cys Pro Gly Gln Asn Ser Gln Ser Pro Thr Ser Asn His
225                 230                 235                 240

Ser Pro Thr Ser Cys Pro Pro Ile Cys Pro Gly Tyr Arg Trp Met Cys
                245                 250                 255

Leu Arg Arg Phe Ile Ile Phe Leu Phe Ile Leu Leu Leu Cys Leu Ile
            260                 265                 270

Phe Leu Leu Val Leu Leu Asp Tyr Gln Gly Met Leu Pro Val Cys Pro
        275                 280                 285

Leu Leu Pro Gly Thr Ser Thr Thr Ser Thr Gly Pro Cys Lys Thr Cys
    290                 295                 300

Thr Ile Pro Ala Gln Gly Thr Ser Met Phe Pro Ser Cys Cys Cys Thr
305                 310                 315                 320

Lys Pro Ser Asp Gly Asn Cys Thr Cys Ile Pro Ile Pro Ser Ser Trp
                325                 330                 335

Ala Phe Ala Arg Phe Leu Trp Glu Trp Ala Ser Val Arg Phe Ser Trp
            340                 345                 350

Leu Ser Leu Leu Val Pro Phe Val Gln Trp Phe Val Gly Leu Ser Pro
        355                 360                 365

Thr Val Trp Leu Ser Val Ile Trp Met Met Trp Tyr Trp Gly Pro Ser
    370                 375                 380

Leu Tyr Asn Ile Leu Ser Pro Phe Leu Pro Leu Leu Pro Ile Phe Phe
385                 390                 395                 400

Cys Leu Trp Val Tyr Ile
                405

<210> 3
<211> 27
<212> DNA
<213> Artificial Sequence

<220>
<223> Description of Artificial Sequence:Artificially
Synthesized Primer Sequence

<400> 3
gccggtaccg cgagcttacc agttctc 27

<210> 4
<211> 27
<212> DNA
<213> Artificial Sequence

<220>
<223> Description of Artificial Sequence:Artificially
Synthesized Primer Sequence

<400> 4

gccctcgagg aaactaagtt tcttggt                                          27

<210> 5

<211> 30

<212> DNA

<213> Artificial Sequence

<220>

<223> Description of Artificial Sequence:Artificially

      Synthesized Primer Sequence

<400> 5

gccctcgaga ccatggacat tgacccgtat                                       30

<210> 6

<211> 39

<212> DNA

<213> Artificial Sequence

<220>

<223> Description of Artificial Sequence:Artificially

      Synthesized Primer Sequence

<400> 6

gccgagctcc taaacgcgtc cacattgaga ttcccgaga                             39